# EUROPEAN PATENT SPECIFICATION

(11) **EP 4 194 181 B1**
(45) Date of publication and mention of the grant of the patent: **20.08.2025**
(21) Application number: 21854307.2
(22) Date of filing: 07.04.2021
(51) Int. Cl.: B29C 64/205, B29C 64/106, B29C 64/393, B29C 64/364, B33Y 70/00, B33Y 30/00, B33Y 10/00, B33Y 50/02, B33Y 40/00, B29C 64/112, B29C 64/209, B05B 17/06, B05B 17/00, B05B 7/08, C12M 1/26

(54) **LEAKAGE PREVENTION SYSTEM FOR DOWNWARD SPRAYING-TYPE NEBULIZER FOR STACKING BIOMATERIAL OF BIO-3D PRINTER**
LECKAGEVERHINDERUNGSSYSTEM FÜR ABWÄRTSSPRÜHZERSTÄUBER ZUM STAPELN VON BIOMATERIAL EINES BIO-3D-DRUCKERS
SYSTÈME DE PRÉVENTION DE FUITE POUR NÉBULISATEUR DE TYPE À PULVÉRISATION VERS LE BAS POUR EMPILER UN BIOMATÉRIAU DE BIO-IMPRIMANTE 3D

(30) Priority: 05.08.2020 KR 20200098115
(43) Date of publication of application: 14.06.2023
(73) Proprietor: Clecell Co., Ltd., Seongnam-si, Gyeonggi-do 13605 (KR)
(72) Inventor: KANG, Min Hyuk, Seoul 01816 (KR); CHOI, Jong Eon, Seoul 01416 (KR); JEON, Kyoung Whee, Seoul 02805 (KR)
(74) Representative: Galbaian S.Coop.
(86) International application number: PCT/KR2021/004314
(87) International publication number: WO 2022/030722

(56) References cited:
- EP-A1- 3 517 273
- WO-A1-2017/071388
- CN-A- 105 835 367
- CN-A- 107 418 872
- JP-A- 2019 510 879
- JP-B1- 6 594 582
- KR-A- 20160 033 126
- KR-B1- 101 805 774
- KR-B1- 101 992 625
- US-A1- 2012 154 482

## Description

### BACKGROUND

### Technical Field

The present disclosure relates to a technology for a bio-3D printer, particularly to a technology for preventing unexpected leakage of a biomaterial from a nebulizer due to gravity in a 3D printing device including a nebulizer that downwardly sprays the biomaterial into the form of mist.

### Related Art

A 3D printer includes a frame constituting the XYZ axis, and a nozzle capable of spraying and stacking a liquid or powder material in a flat form so as to form a three dimensional form. The 3D printer encompasses, for example, Cartesian-, Mendel-, Delta-, Core XY-style 3D printers, etc. depending on a printing method. In the Cartesian-style 3D printer, a bed moves in the X and Y axes, and a nozzle moves in the Z axis direction to implement 3D printing. In the Mendel-style 3D printer, a bed moves in the Z axis direction and a nozzle moves in the X, Y axes to implement 3D printing. In the Delta-style 3D printer, a nozzle moves in the X, Y, Z axes to implement 3D printing. The Core XY-style 3D printer, a nozzle moves in the X, Y axis directions by a belt connected by two motors and a bed moves in the Z direction. Recently, the Core XY-style 3D printer, which is capable of controlling the position of the nozzle most precisely, is widely used.

A bio-3D printer is a device that has been structurally modified so as to three-dimensionally create a living tissue or an organ by discharging a biomaterial through the nozzle of the 3D printer.

This bio-3D printer includes a dispenser-type for discharging the biomaterial. The bio-3D printer fills the nozzle with a viscous biomaterial such as collagen, gelatin, etc. This adopts a method that connects a pneumatic system to the nozzle filled with the viscous biomaterial to discharge the biomaterial while pushing it out.

An example of this bio-3D printer is disclosed in Korea Patent No. 1828345. Meanwhile, when the viscosity is low depending on the type of biomaterials employed in the bio-3D printer, the dispenser-type nozzle as described above cannot be used. When the fluidity of the biomaterial resulting from its low viscosity is excessively high, the dispenser-type nozzle adopting an extrusion method to squeeze the material cannot be used because it is difficult to control the fluid. In this case, it is necessary to use a valve-type dispenser which controls and sprays a small amount of biomaterial in s droplet form. When outputting a biomaterial with high fluidity, a method of layered cross-liking or mixed crosslinking can be used while spraying the biomaterial onto a substrate in a mist form by employing a nebulizer, for example, as disclosed in US Patent Publication No. 2011-0212501.

However, when employing the nebulizer as the above-mentioned, the nebulizer must be configured to downwardly spray the biomaterial toward the substrate. Most biomaterials used in the bio-3D printer are expensive. However, in the case of a downward spraying-type nebulizer, there is a problem that the biomaterial accommodated in the nebulizer unexpectedly leaks downwards due to gravity while the output thereof is stopped. In general, it takes 30 minutes to 60 minutes to output the biomaterial form the bio-3D printer. In this case, when conducting a study in the order of output --> incubation --> output --> incubation --> observation, it may take more than 2 weeks to output the biomaterial. In this process, when the biomaterial accommodated in the nebulizer leaks unexpectedly, there is a problem that not only economic loss but also fatal errors may occur.

EP 3 517 273 A1 discloses a bio-3D printer that dispenses biomaterial and also medical adhesive. In one embodiment the bio-3D printer comprises a leakage prevention systema for the medical adhesive.

### SUMMARY

### Technical Solution

The present disclosure is contrived to solve the aforementioned problem, providing a bio-3D printer comprising an output unit constituted by a leakage prevention system for a downward spraying-type nebulizer for stacking a biomaterial of the bio-3D printer including a nebulizer that is configured to spray the biomaterial downwards so as to prevent the biomaterial from leaking.

### Technical Solution

In order to achieve the object as mentioned above, provided is a bio-3D printer comprising an output unit constituted by a leakage prevention system for a downward spraying-type nebulizer for stacking a biomaterial of the bio-3D printer according to the present disclosure, wherein the system constituting an output unit of the bio-3D printer includes:
a nebulizer that downwardly sprays a biomaterial into a form of mist toward a substrate, the nebulizer including a space accommodating the biomaterial at an upper portion thereof, and having a vibrating membrane installed at a lower portion thereof;
a vacuum cap that is detachably installed on a upper portion of the nebulizer;
a distributor that is connected to the vacuum cap with a hose;
a vacuum generating module that is connected to the distributor with a hose;
a compressor that generates an air pressure in the vacuum generating module;
a controller that is electrically connected to the vacuum generating module and the nebulizer, controlling an output pressure of the vacuum generating module and an action of the nebulizer, wherein the vibrating membrane of the nebulizer vibrates at a high frequency in response to a signal of the controller so that the biomaterial accommodated in the nebulizer is divided into a mist form and sprayed downwards; and
a moisture removal filter installed between the vacuum cap and the distributor or between the distributor and the vacuum generating module for preventing the vacuum generating module from being damaged by back flowed biomaterial.

### Advantageous Effects

The leakage prevention system for a downward spraying-type nebulizer for stacking a biomaterial of the bio-3D printer according to the present disclosure is suitable to apply an air pressure below atmospheric pressure within a preset range to the nebulizer during a time when the nebulizer is not operated, so as to provide a prevention effect of leakage of biomaterial due to gravity.

### BRIEF DESCRIPTION OF THE DRAWINGS

FIG. 1 is a structural view of a system according to a preferred embodiment of the present disclosure.
FIG. 2 is an exploded perspective view of the configuration in the vicinity of a nebulizer.
FIG. 3 is an enlarged view of the configuration in the vicinity of the nebulizer as shown in FIG. 1.
FIG. 4 is a partial cross-sectional view taken long line IV - VI as shown in FIG. 3.

### DETAILED DESCRIPTION

### Best Mode

Hereinafter, the preferred embodiment of the present disclosure will be described in detail with reference to the accompanying drawings.

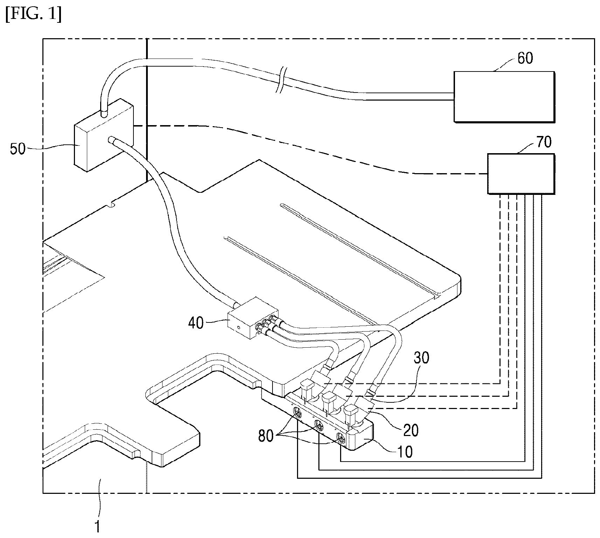

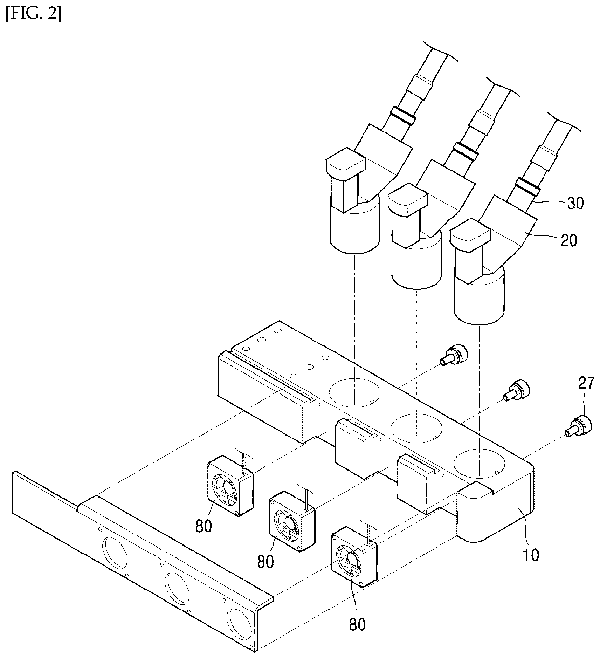

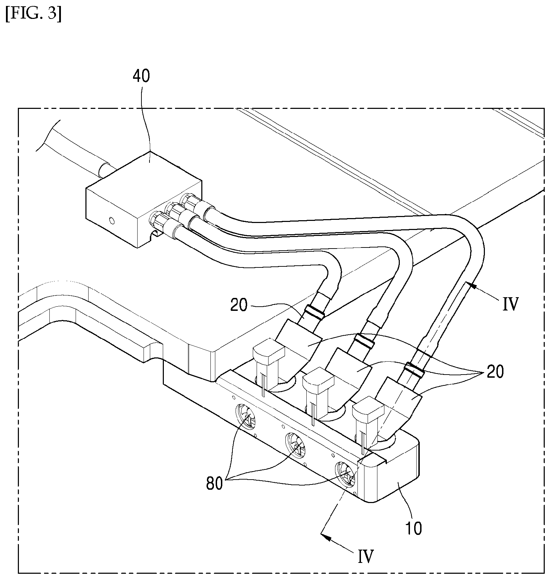

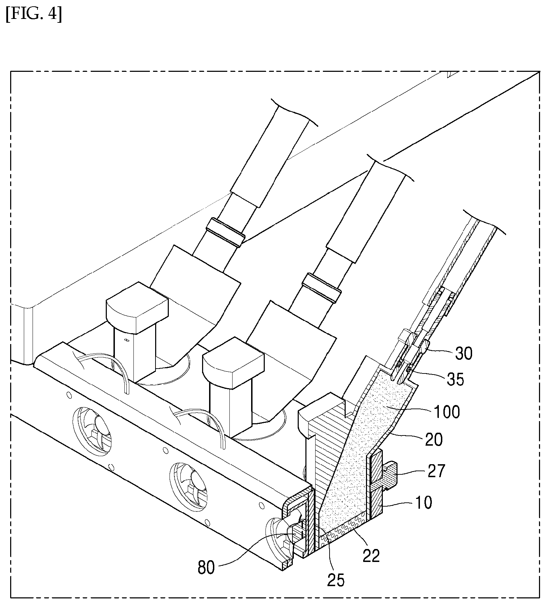

FIG. 1 is a structural view of a system according to a preferred embodiment of the present disclosure. FIG. 2 is an exploded perspective view of the configuration in the vicinity of a nebulizer. FIG. 3 is an enlarged view of the configuration in the vicinity of the nebulizer as shown in FIG. 1. FIG. 4 is a partial cross-sectional view taken long line IV - VI as shown in FIG. 3.

Referring to FIGS. 1 to 4, a leakage prevention system for a downward spraying-type nebulizer for stacking a biomaterial of a bio-3D printer (hereinafter, referred to as "leakage prevention system for a nebulizer") includes a cartridge 10, a nebulizer 20, a vacuum cap 30, a distributor 40, a vacuum generating module 50, a compressor 60, a controller 70 and a drying fan 80.

The leakage prevention system for a nebulizer is a system constituting an output unit of the bio-3D printer.

The nebulizer cartridge 10 is fixed to a housing 1 of the bio-3D printer. The nebulizer cartridge 10 is a rod-shaped structure, including a plurality of installation holes 25 formed to penetrate the cartridge in a direction perpendicular to the ground so as to detachably install the plurality of nebulizers 20. The drying fan 80 blowing air to promote drying of the biomaterial 100 sprayed from the nebulizer 20 may be installed on a side surface of the nebulizer cartridge 10.

The nebulizer 20 is a device that downwardly sprays the biomaterial 100 into a form of mist toward a substrate. The nebulizer 20 is installed in a state partially accommodated in the installation hole 25. The nebulizer 20 may be firmly fixed to the nebulizer cartridge 10 with a fixing member 27 to be screwed in a lateral direction of the installation hole 25. The nebulizer 20 includes a space accommodating the biomaterial 100 at an upper portion thereof. The nebulizer has a vibrating membrane 22 installed at a lower portion thereof. The nebulizer 20 employed in the present disclosure is a vibrating membrane type. The vibrating membrane 22 vibrates at a high frequency in response to a signal of the controller 70 to be described later so that the biomaterial 100 accommodated in the nebulizer 20 is divided into a mist form and sprayed downwards. The principal of the nebulizer 20 employed in the present disclosure is the same as that of a known vibrating membrane-type nebulizer. A particular size of the biomaterial 100 sprayed in the nebulizer 20 is preferably 5µm or less. The nebulizer 20 may be provided in plurality.

The vacuum cap 30 is detachably installed on the upper portion of the nebulizer 20. The vacuum cap 30 is installed to the nebulizer 20 to have a coupling force to the extent of being installed or detached by a person's hand. More particularly, a force applied for coupling or detaching the vacuum cap 30 and the nebulizer 20 is preferably 20N to 100N.

The vacuum cap 30 is connected to the distributor 40 to be described later with a hose. An O-ring 35 is connected to the vacuum cap 30. The O-ring 35 is installed at a contact portion of the vacuum cap 30 and the nebulizer 20, preventing a gap between the vacuum cap 30 and the nebulizer 20.

The distributor 40 is connected to the vacuum cap 30 through a hose. The distributor 40 evenly distributes an air pressure of the negative pressure generated in the vacuum generating module 50 to be described later, performing a role to act this on each of the nebulizers 20. The distributor 40 may adopt a known structure. An air pressure output from the vacuum generating module 50 is preferably - 1.5KPa to 0KPa. When an output air pressure of the vacuum generating module 50 is less than -1.5KPa, there is a problem that the biomaterial 100 accommodated in the nebulizer 20 may flow backwards toward the distributor 40. When an output air pressure of the vacuum generating module 50 is more than 0KPa, there is a problem that the biomaterial 100 accommodated in the nebulizer 20 cannot be prevented from leaking downwards.

The vacuum generating module 50 is connected to the distributor 40 with a hose. The vacuum generating module 50 is a device that generates an air pressure lower than atmospheric pressure in a specific branch pipe using the principal of the sprayer. The vacuum generating module 50 may be configured by employing a known vacuum generating module. The vacuum generating module 50 is electrically connected to the controller 70 to be described later, allowing variably adjusting a pressure generated by a signal of the controller 70.

The compressor 60 is a device for acting an air pressure on the vacuum generating module 50. As the air pressure generated in the compressor 60 is discharged to the outside through the vacuum generating module 50, an air pressure lower than atmospheric pressure is formed in a specific portion of the vacuum generating module 50.

A moisture removal filter (not illustrated) is installed between the vacuum cap 30 and the distributor 40 or between the distributor 40 and the vacuum generating module 50. When a pressure generated in the vacuum generating module 50 exceeds an appropriate range and a greater negative pressure than necessary for the biomaterial 100 is generated, the biomaterial 100 may flow backwards from the nebulizer 20 toward the vacuum generating module 50. In this case, the moisture removal filter performs a role to prevent the vacuum generating module 50 from being damaged by the back flowed biomaterial 100.

The controller 70 is electrically connected to the vacuum generating module 50 and the nebulizer 20. The controller 70 controls an output pressure of the vacuum generating module 50. Further, the controller 70 controls the operation of the nebulizer 20.

The drying fan 80 is arranged in the vicinity of the biomaterial 100 sprayed onto the substrate. The drying fan 80 is a device for blowing air to promote drying of the biomaterial 100 sprayed onto the substrate. The drying fan 80 may be controlled by the controller 70. The drying fan 80 is preferably installed to the nebulizer cartridge 10. An axial fan or a sirocco fan may be adopted as the drying fan 80. The drying fan 80 is configured to suck air from a lateral direction of the nebulizer 20 and to discharge it to a downward direction of the nebulizer 20.

The effect of the leaking prevention system for a downward spraying-type nebulizer for stacking a biomaterial of a bio-3D printer including the above-described components will be described in detail according to the operation sequence of the nebulizer.

Firstly, the nebulizer 20 is filled with the biomaterial 100, and then the upper portion thereof is sealed with a vacuum cap 30. The vacuum cap 30 is connected to the distributor 40 with a hose. Further, the distributor 40 is connected to the vacuum generating module 50 with another hose. The compressor 60 and the vacuum generating module 50 are connected by a hose.

In this state, the nebulizer 20 is operated by a signal of the controller 70. As the vibrating membrane 22 of the nebulizer 20 vibrates at a high frequency, the biomaterial 100 is sprayed to the downward direction of the nebulizer 20. After maintaining such a spraying for a predetermined time, the nebulizer 20 is stopped. The compressor 60 is always operated at the same time of turning the bio-3D printer on, regardless the operation of the nebulizer 20. Accordingly, an air pressure lower than atmospheric pressure is generated in the vacuum generating module 50, and a pressure below atmospheric pressure within a certain range acts on each of the nebulizers 20 through the distributor 40. The biomaterial 100 accommodated in the nebulizer 20 may leak to the downward direction of the nebulizer 20 through a pore of the vibrating membrane 22 by gravity even when the vibrating membrane 22 does not vibrate. However, the biomaterial 100 accommodated in the nebulizer 20 does not leak from the nebulizer 20 by a pressure below atmospheric pressure generated in the vacuum generating module 50.

When the nebulizer 20 is operated again by the controller 70, the nebulizer 20 performs a normal output operation of the biomaterial 100 by vibration of the vibrating membrane 22.

As described above, the leakage prevention for a downward spraying-type for stacking a biomaterial of a bio-3D printer according to the present disclosure is configured to apply an air pressure below atmospheric pressure within a preset range to the nebulizer, providing a prevention effect of leakage of biomaterial due to gravity during a time when the nebulizer is not operated.

### Description of Embodiments

In order to achieve the object as mentioned above, provided is a bio-3D printer comprising an output unit constituted by a leaking prevention system for a downward spraying-type nebulizer for stacking a biomaterial of the bio-3D printer according to the present disclosure, wherein the system constituting an output unit of the bio-3D printer includes:
a nebulizer that downwardly sprays a biomaterial into a form of mist toward a substrate, the nebulizer including a space accommodating the biomaterial at an upper portion thereof, and having a vibrating membrane (22) installed at a lower portion thereof;
a vacuum cap that is detachably installed on a upper portion of the nebulizer;
a distributor that is connected to the vacuum cap with a hose;
a vacuum generating module that is connected to the distributor with a hose;
a compressor that generates an air pressure in the vacuum generating module;
a controller that is electrically connected to the vacuum generating module and the nebulizer, controlling an output pressure of the vacuum generating module and an action of the nebulizer, wherein the vibrating membrane of the nebulizer vibrates at a high frequency in response to a signal of the controller so that the biomaterial accommodated in the nebulizer is divided into a mist form and sprayed downwards; and
a moisture removal filter installed between the vacuum cap and the distributor or between the distributor and the vacuum generating module for preventing the vacuum generating module from being damaged by back flowed biomaterial.

An air pressure output from the vacuum generating module is preferably -1.5KPa to 0KPa.

The vacuum cap preferably has an O-ring at a portion in contact with the nebulizer.

The leaking prevention system for a downward spraying-type nebulizer for stacking a biomaterial of a bio-3D printer preferably includes a drying fan that is arranged in the vicinity of the nebulizer, blowing air to promote drying of the biomaterial sprayed onto the substrate.

## Claims

1. A bio-3D printer comprising an output unit constituted by a leakage prevention system for a downward spraying-type nebulizer for stacking a biomaterial of the bio-3D printer, **characterized in that**
the leakage prevention system comprises:
a nebulizer (20) that downwardly sprays a biomaterial (100) into a form of mist toward a substrate, the nebulizer (20) including a space accommodating the biomaterial (100) at an upper portion thereof, and having a vibrating membrane (22) installed at a lower portion thereof;
a vacuum cap (30) that is detachably installed on a upper portion of the nebulizer (20);
a distributor (40) that is connected to the vacuum cap (30) with a hose;
a vacuum generating module (50) that is connected to the distributor (40) with a hose;
a compressor (60) that generates an air pressure in the vacuum generating module (50);
a controller (70) that is electrically connected to the vacuum generating module (50) and the nebulizer (20), controlling an output pressure of the vacuum generating module (50) and an operation of the nebulizer (20), wherein the vibrating membrane (22) of the nebulizer (20) vibrates at a high frequency in response to a signal of the controller (70) so that the biomaterial (100) accommodated in the nebulizer (20) is divided into a mist form and sprayed downwards; and
a moisture removal filter installed between the vacuum cap (30) and the distributor (40) or between the distributor (40) and the vacuum generating module (50) for preventing the vacuum generating module (50) from being damaged by back flowed biomaterial (100).

2. The bio-3D printer of claim 1, wherein
the air pressure is -1.5KPa to 0KPa.

3. The bio-3D printer of claim 1, wherein
the vacuum cap (30) has an O-ring (35) at a portion in contact with the nebulizer (20).

4. The bio-3D printer of claim 1, further comprising:
a drying fan (80) that is arranged in the vicinity of the nebulizer (20), blowing air to promote drying of the biomaterial sprayed onto the substrate.

## Patentansprüche

1. Bio-3D-Drucker mit einer Ausgabeeinheit, bestehend aus einem Leckageverhinderungssystem für Abwärtssprühzerstäuber zum Stapeln von Biomaterial des Bio-3D-Druckers, **dadurch gekennzeichnet, dass** das Leckageverhinderungssystem Folgendes umfasst:
einen Sprühzerstäuber (20), der ein Biomaterial (100) in Form eines Nebels nach unten in Richtung eines Substrats sprüht, wobei der Sprühzerstäuber (20) in seinem oberen Bereich einen Raum zur Aufnahme des Biomaterials (100) aufweist und eine in seinem unteren Bereich angeordnete vibrierende Membran (22) aufweist;
eine Vakuumkappe (30), die abnehmbar im oberen Bereich des Sprühzerstäubers (20) angeordnet ist;
einen Verteiler (40), der über einen Schlauch mit der Vakuumkappe (30) verbunden ist;
ein Vakuumerzeugungsmodul (50), das über einen Schlauch mit dem Verteiler (40) verbunden ist;
einen Kompressor (60), der in dem Vakuumerzeugungsmodul (50) einen Luftdruck erzeugt;
eine Steuerung (70), die mit dem Vakuumerzeugungsmodul (50) und dem Sprühzerstäuber (20) elektrisch verbunden ist und den Austrittsdruck des Vakuumerzeugungsmoduls (50) und die Funktion des Sprühzerstäubers (20) steuert, wobei die vibrierende Membran (22) des Sprühzerstäubers (20) als Reaktion auf ein Signal der Steuerung (70) mit einer hohen Frequenz vibriert, sodass das in dem Sprühzerstäuber (20) enthaltene Biomaterial (100) in eine Nebelform aufgespalten und nach unten versprüht wird; und
einen Entfeuchtungsfilter, der zwischen der Vakuumkappe (30) und dem Verteiler (40) oder zwischen dem Verteiler (40) und dem Vakuumerzeugungsmodul (50) angeordnet ist, um eine Beschädigung des Vakuumerzeugungsmoduls (50) durch zurückströmendes Biomaterial (100) zu verhindern.

2. Bio-3D-Drucker nach Anspruch 1, wobei
der Luftdruck zwischen -1,5 kPa und 0 kPa liegt.

3. Bio-3D-Drucker nach Anspruch 1, wobei
die Vakuumkappe (30) in einem Bereich in Kontakt mit dem Sprühzerstäuber (20) einen O-Ring (35) aufweist.

4. Bio-3D-Drucker nach Anspruch 1, ferner umfassend:
ein Trocknungsgebläse (80), das in der Nähe des Sprühzerstäubers (20) angeordnet ist und zur beschleunigten Trocknung des auf das Substrat gesprühten Biomaterials Luft ausbläst.

## Revendications

1. Bio-imprimante 3D comprenant une unité de sortie constituée par un système de prévention de fuite pour un nébulisateur de type à pulvérisation vers le bas pour empiler un biomatériau de la bio-imprimante 3D, **caractérisée en ce que** le système de prévention de fuite comprend:
un nébulisateur (20) qui pulvérise vers le bas un biomatériau (100) sous forme de brouillard vers un substrat, le nébulisateur (20) comportant un espace logeant le biomatériau (100) dans une portion supérieure de celui-ci, et ayant une membrane vibrante (22) installée dans une portion inférieure de celui-ci;
un capuchon sous vide (30) qui est installé de manière amovible sur une portion supérieure du nébulisateur (20);
un distributeur (40) qui est relié au capuchon sous vide (30) avec un tuyau ;
un module de génération de vide (50) qui est relié au distributeur (40) avec un tuyau;
un compresseur (60) qui génère une pression d'air dans le module de génération de vide (50);
un contrôleur (70) qui est électriquement relié au module de génération de vide (50) et au nébulisateur (20), contrôlant une pression de sortie du module de génération de vide (50) et un fonctionnement du nébulisateur (20), dans laquelle la membrane vibrante (22) du nébulisateur (20) vibre à une haute fréquence en réponse à un signal du contrôleur (70) de telle sorte que le biomatériau (100) logé dans le nébulisateur (20) est divisé sous forme d'un brouillard et pulvérisé vers le bas; et
un filtre d'élimination d'humidité installé entre le capuchon sous vide (30) et le distributeur (40) ou entre le distributeur (40) et le module de génération de vide (50) pour empêcher que le module de génération de vide (50) ne soit endommagé par un écoulement de retour du biomatériau (100).

2. Bio-imprimante 3D selon la revendication 1, dans laquelle
la pression d'air est de -1,5 KPa à 0 KPa.

3. Bio-imprimante 3D selon la revendication 1, dans laquelle
le capuchon sous vide (30) présente un joint torique (35) dans une portion en contact avec le nébulisateur (20).

4. Bio-imprimante 3D selon la revendication 1, comprenant en outre:
un ventilateur de séchage (80) qui est disposé au voisinage du nébulisateur (20), soufflant de l'air pour favoriser le séchage du biomatériau pulvérisé sur le substrat.
